# EUROPEAN PATENT SPECIFICATION

(11) **EP 1 601 288 B1**
(45) Date of publication and mention of the grant of the patent: **09.05.2012**
(21) Application number: 03716312.8
(22) Date of filing: 05.03.2003
(51) Int. Cl.: A61B 6/00, A61N 5/06, A61B 18/22

(54) **CATHETER PROBE ARRANGEMENT FOR TISSUE ANALYSIS BY RADIANT ENERGY DELIVERY AND RADIANT ENERGY COLLECTION**
KATHETERSONDENANORDNUNG FÜR GEWEBEANALYSE MITTELS STRAHLUNGSENERGIEABGABE UND STRAHLUNGSENERGIESAMMLUNG
AGENCEMENT DE SONDE DE CATHETER POUR L'ANALYSE DE TISSUS PAR APPLICATION D'ENERGIE RAYONNANTE ET PAR COLLECTE D'ENERGIE RAYONNANTE

(43) Date of publication of application: 07.12.2005
(60) Divisional application: 10185897.5
(73) Proprietor: InfraReDx, Inc., Cambridge, MA 02140 (US)
(72) Inventor: FURNISH, Simon, Louisville, KY 40202 (US)
(74) Representative: Hutchinson, Glenn Stanley
(86) International application number: PCT/US2003/006735
(87) International publication number: WO 2004/078044

(56) References cited:
- EP-A- 0 947 221
- EP-A- 1 075 821
- WO-A2-02/096484
- DE-A1- 4 326 037
- US-A- 4 195 904
- US-A- 5 582 170
- US-A- 5 836 941
- US-A1- 2002 183 622
- US-A1- 2002 183 623
- US-B1- 6 416 234
- US-B1- 6 577 891

## Description

### Prior Art

The sensing and treating of various tissue characteristics in the in vivo intravascular environment is desirous for many reasons yet difficult because it is a very harsh environment in which to conduct such analysis or treatment. The presence of blood and its constituents such as cholesterol may effect scattering and absorption of energy signals transmitted within an origan. Diagnosis and treatment of various tissues within the human body using an in vivo probe necessitates adaptive characteristics for that probe when it is inserted into a mammalian body organ.

US 2002/0183623 discloses a multi-path optical catheter in which first and second optical-redirectors mounted on a catheter couple radiation to a target along separate first and second paths. This document forms a pre-characterising portion of the claims appended hereto.

EP 1075821 discloses a laser or radiation apparatus comprising a long and slender main body, a rotating shaft and multiple reflective mirrors installed on the rotating shaft which are located in different positions along the longitudinal and circumferential directions of the rotating shaft.

DE 4326037 discloses a laser device having at least two laser beams for cauterising cartilage plates adjacent a vertebrae.

It is an object of the present invention to provide a probe for insertion within a mammalian body which overcomes the disadvantages of the prior art.

It is a further object of the present invention to provide a minimally invasive device for light energy transmitting (i.e. infrared through ultraviolet) diagnosis and treatment of mammalian tissue through the use of endoscopes, catheters and other minimally invasive devices.

It is still yet a further object of the present invention to provide optical probe tip arrangement which facilitates optimum delivery and retrieval of energy signals within the human body tissue.

### Brief Summary of the invention

The invention is as disclosed in the appended set of claims.

The present invention provides apparatus to analyze body tissue using an energy spectrum analysis distributed and received by an elongated probe introducable through a catheter into that body tissue. That introduction of the body probe may be done through an endoscope, or other catheter-like devices for such energy diagnosis and treatment of tissue. That energy analysis and treatment may include near infrared (NIR) reflectance spectroscopy, Raman spectroscopy, fluorescence spectroscopy, photo- dynamic drug activation, photonic ablation or thermal treatments, and optical coherence tomography.

According to the present invention, there is provided a catheter tip apparatus arranged in a catheter for the delivery and collection of an energy signal to permit subsequent light energy beam analysis of body tissue by the collected signal, characterised by: an elongated housing supporting a first reflective surface and a second reflective surface, said first reflective surface and said second reflective surface being longitudinally spaced apart from one another; a first flexible, elongated energy bearing delivery fiber having a distalmost end arranged adjacent said first reflective surface; a second flexible, elongated energy bearing collection fiber having a distalmost end arranged adjacent said second reflective surface; and said housing rotatably supported within a flexible catheter sheath for insertion of said catheter into a mammalian body for tissue analysis thereof.

The probe of the present invention comprises an elongated, generally cylindrically shaped housing having a. first or distal end and a second, or proximal end. The proximal end has a stem thereon of reduced diameter from the diameter of the distalmost portion thereof. An elongated groove is arranged to extend from the proximal end of the stem through towards the distal end of the housing. The groove is disposed through only one side of the housing, and has an arrangement of angled shoulders therein for providing snug receipt of the collector and the delivery fiber arrangement.

The collector fiber arrangement in this particular embodiment includes an elongated flexible collection fiber having a distal end to which a reflector or reflective surface (i.e., a mirror member) is attached. The collection fiber has an outer buffer such as a sheath for protection of the fiber and to minimize stray radiation therefrom. A mirror member has an angularly disposed reflective surface thereon. The delivery reflector (or mirror member) is attached to an optical delivery fiber which is enclosed similarly by an outer buffer such as a sheath for protection of the fiber and for minimization of light leakage. The delivery reflector or mirror member has an angled reflective surface thereon. The collection fiber and reflector and the delivery fiber and reflector jointly mate within the elongated receiving groove within the stem and tip housing. The elongated groove is preferably shaped to effect accurate positioning of the respective reflectors or mirror members therein, so as to emit radiation from the delivery reflector (or mirror member) and receive radiation reflected back from a body tissue sample in the collection reflector (or mirror member). Once the collection and delivery fibers are within the housing, those joint fibers may be inserted within an elongated catheter shaft or rotatable coil as will be described hereinbelow.

A further embodiment of the present invention is disclosed by a elongated support frame having a proximal end and a distal end. The proximal end includes and upstanding portion through which a collection fiber channel is arranged (i.e., molded, drilled, machined). A rectilinearly-shaped holding pocket is arranged distal of the first upstanding member and is arranged to receive a reflective mirrored surface for example, a collection prism, thereon. A midblock portion is arranged centrally in the support frame and has a delivery fiber channel arranged therewithin, the delivery fiber channel extending parallel and adjacent the collection fiber channel. A second holding pocket is similarly arranged adjacent the delivery fiber channel for receipt of a second reflector such as a reflective mirrored surface, such as for example, a mirror member, having a mirrored surface thereon. The holding pockets are constructed so as to accurately receive and align the respective first and second mirror members to the desired angle for the desired photon delivery and photon collection from a target body tissue. The elongated support frame is arranged within an elongated housing, having an elongated channel for receipt thereof. A delivery fiber and a collection fiber would be inserted within their respective channels and the respective reflectors (i.e. mirror members) would be secured (i.e. affixed by adhesive) within their respective holding pockets.

A further embodiment of the optical probe arrangement of the present invention is characterized by a generally cylindrically shaped frame member having a stepped down stem portion on its proximal end thereof. The frame member is arranged so as to define a series of distal step portions, the first portion of which is arranged to receive a collection reflector (i.e. mirror member or reflective surface) and second stepped portion receives a delivery reflector (i.e. mirror member or reflective surface). A bore or channel is arranged through the frame member for servicing each particular reflector. Each respective channel receives an optical fiber which is arranged to abut one surface of its respective reflector. The distal cover may be arranged so as to mate over the respective collection reflector and the delivery reflector while having a slot for passage of a photonic signal therethrough. The delivery reflector is thus permitted to emit photonic radiation and the collection reflector is permitted to receive photonic radiation when the frame member is assembled with the cover, and the stem is attached in holding the particular fiber, and those fibers are inserted within a hollow torqueable transmission shaft such as a counterwound multifilar drive shaft coil arrangement proximal of that frame member.

A further embodiment of the probe arrangement is shown by an elongated frame member having a proximal end and a distal end. The proximal end of that frame member is arranged so as to define an angled reflective surface thereat, the distal end of that frame member having a second reflective surface angularly disposed thereon with an oval end cap thereon. A bore is arranged through the proximal end of the elongated frame member to define a receiving channel for a delivery fiber to be inserted therewithin. The entire elongated frame member is inserted into an elongated cylindrically shaped housing having a proximal end and a distal end. The proximal end of the housing has a stem of stepped down diameter, which stem encloses an energy delivery fiber and an energy collection fiber. The stem of the housing is arranged to receive the collection fiber which is arranged to receive reflective signals from its reflector surface arranged onto the elongated frame member. The housing has an opening arranged longitudinally therein, so as to receive the elongated frame member. The housing also has a slot cut through a side portion thereof so as to permit the energy signal to be delivered and received by the respective reflective surfaces contained therewithin. The end cap on the distalmost end of the elongated frame member abuts the distalmost end of the elongated housing to define a smooth continuous surface therearound.

A further embodiment of the present invention resides in a one piece housing of generally cylindrical shape having a proximal portion of stepped down diameter defining a stem. A first reflective collection surface is disposed at a particularly desired angle adjacent the stem of the elongated housing, and a second reflective surface is arranged into the elongated housing adjacent its distalmost end. A first bore is arranged through the housing through the stem so as to receive a collection fiber. A second bore is also arranged through the elongated housing so as to support a delivery fiber therethrough. The stem may be received in a pair of hollow, flexible, counterwound coils which function as a torqable transmission shaft which is secured to the stem and surrounds the delivery and collection fibers received therethrough. The first and second reflective surfaces arranged in the housing, in this embodiment, may be skewed (non-parallel or curvilinear) so as to emit and receive signals from any particular direction with respect to its adjacent reflective surface.

A yet further embodiment of the present invention relates to a method of constructing a catheter tip arrangement for support of a plurality of optical fibers, which support construction permits minimization of component size and adaptive angularity of reflection of the delivery and collection beams. Such a support may be accomplished by micromachining construction where additive processing such as for example: plating, sputtering, vapor deposition, and subtractive processing such as for example: etching, laser cutting and ablation, permits finite adjustment to the dimensions. The support comprises a base upon which an arrangement of parallel bosses are "grown", the bosses defining between them, a pair of parallel slots into which a delivery and a collection fiber may be mated. A mirror surface and support struts are spaced at the distalmost location of the fibers, which mirror surface may be curved or manipulably bent to the desired angle for maximizing optical analysis and tissue treatment thereby. This embodiment contemplates the use of index-matching fluids added to any gap between a catheter sheath surrounding the fibers to reduce any back reflections from the interior of the protective sheath/transmission window.

The invention thus comprises a catheter tip apparatus arranged in a catheter for the delivery and collection of a light energy signal to permit subsequent computerized analysis of body tissue by the collected signal. The apparatus comprises an elongated housing member supporting a first reflective surface and a second reflective surface. The first reflective surface and the second reflective surface are longitudinally spaced apart from one
another. A first flexible, elongated, light energy bearing delivery fiber has a distalmost end arranged adjacent the first reflective surface. A second flexible, elongated energy bearing collection fiber has a distalmost end arranged adjacent the second reflective surface. The housing member is rotatably supported on a flexible catheter sheath for insertion of the catheter into a mammalian body for tissue analysis thereof The housing may comprise a frame member having a slot arranged therein for receipt and alignment of the first and the second reflective surfaces. The first and the second reflective surfaces may comprise prism members. The slot may have shoulders therein to secure and accurately align the reflective surfaces therein. The housing may have a proximalmost stem portion for receipt into a catheter sheath to permit manipulation of the tip from a proximal location. The housing may be comprised of a frame member having a proximal end and a distal end, with an upstanding proximal block and an upstanding midblock, each block having a holding pocket thereadjacent for receipt of a reflective surface attachable therein. The reflective surface may comprise a prism fixedly attached into the pocket. Each of the upstanding blocks may have a bore therethrough for receipt of one of the energy bearing fibers. The housing may comprise an elongated generally cylindrically shaped frame member with a proximal end and a distal end, the frame member having at
least two steps thereon of decreasing thickness in the distal direction, each of the steps having a reflective surface mounted thereon, the proximal end having a stem portion of reduced diameter, to permit rotative receipt within a tubular catheter sheath. The frame member may have a cover member arranged to mate over the steps and the reflective surfaces of the housing. The cover member may have an axially arranged slot thereon through part of its longitudinal length, the slot being disposed radially adjacent each of the reflective surfaces to permit delivery and reflected collection of an energy beam therethrough. The stem portion may be secured to a multi-layered, elongated coil spring arrangement to permit twisting control of the catheter tip within a mammalian body component. The reflective surfaces may be unitary portions of the housing. The housing has a proximal end and a distal end, and the proximal end may mate with a housing enclosure, the enclosure providing a securement means for the energy collecting fiber and the housing providing a securement means for the energy delivery fiber. The housing enclosure attached to said proximal end of said housing may have a longitudinally directed elongated slot therein, the slot being in radial alignment with the reflective surfaces formed on the housing to permit transmission and collection of radiant energy via the respective reflective surfaces to a computerized analysis system. The housing may comprise a cylindrically shaped member having its first and second reflective surfaces machined thereon, and wherein the first and second reflective surfaces are non-parallel with respect to one another. The first and second fibers may be diametrically oppositely arranged with respect to the longitudinal axis of rotation of the housing, to minimize any undesired motion of the housing during its rotation in a body tissue. The housing may include a reflective surface which is bendable to effect directional change of an energy beam reflecting therefrom. The housing may be made of "accumulation" or "deletion" components defining a fiber alignment slot for miniaturization of the tip.

The present invention also comprises a catheter tip apparatus arranged in a catheter for the delivery and collection of an energy signal to permit subsequent computerized analysis of body tissue by the collected signal. The apparatus comprises an elongated housing having a longitudinal axis of rotation, the housing having a first reflective surface disposed thereon, a second reflective surface disposed on the housing distally of the first reflective surface and in axial alignment therewith; and a first light conductive fiber in light coupled communication with the first reflective surface and a second light conductive fiber in light coupled communication with the second reflective surface, the first light conductive fiber in communication being in communication with a controlled analytical-light generating source and the second light conductive fiber being in communication with a light-collection analysis device. The first reflective surface may be dimensionally larger than the second reflective surface. The first reflective surface may be curvilinear. The first reflective surface may be non-parallel with respect to the second reflective surface. At least one of the first and second reflective surfaces may be spaced apart from the light conductive fibers. The first reflective surface may be disposed radially within and spaced from the perimeter of the housing to permit a spreading of a light beam from the first reflective surface onto the body tissue. An index matching fluid may be arranged between a distal end of the conductive fiber and the reflective surface. The reflective surface may be positioned in a holding pocket arranged in the housing. The reflective surface may be comprised of a mirrored surface. The holding pocket may be utilized to align the reflective surface with respect to the housing. The conductive light fibers may be each individually arranged within a bore disposed within the housing. The light delivery fibers may be equally diametrically opposed about the axis of rotation of the housing to provide balance thereto and minimize eccentricity during rotation thereof.

The invention may also comprise a catheter tip apparatus arranged in a catheter for the delivery and collection of an energy signal to permit subsequent computerized analysis of body tissue by the collected signal, the apparatus comprising an elongated housing having a longitudinal axis of rotation, the housing having a first reflective surface disposed thereon, a second reflective surface disposed on the housing distally of the first reflective surface and in axial alignment therewith. A first light conductive fiber may be in light-coupled communication with the first reflective surface and a second light conductive fiber in light-coupled communication with the second reflective surface, the first light conductive fiber in communication being in communication with a controlled analytical-light generating source and the second light conductive fiber being in communication with a light-collection analysis device. A curvilinear cover may be arranged to mate over a distal portion of the housing to enclose the reflective surfaces, the cover having at least one opening on an annular surface thereof to permit light delivery to the body tissue, and to permit light collection therethrough upon reflection from the body tissue. At least one of the reflective surfaces may comprise a mirror or polished surface. Each of the light conductive fibers has a distal end arranged within said housing, and the at least one of the light conductive fibers is in abutting relationship with a non-reflective surface of the member containing the reflective surface. At least one of the reflective surfaces may be disposed in a holding pocket. The reflective surface may be secured in the holding pocket by an adhesive.

The invention also comprises a catheter tip apparatus having a first reflective surface and said second reflective surface which are disposed at an angle proportional to the numerical aperture of the first and second foptical ibers, to yield a light beam with adjacent edges that are parallel to one another, to permit a distance independent delivery reflector-collector reflector separation.

The invention may also comprise a catheter tip apparatus arranged in a catheter for the delivery and collection of an energy signal to permit subsequent computerized analysis of body tissue by the collected signal, comprising an optically transparent sheath enclosed elongated housing having a longitudinal axis of rotation, the housing having a first reflective surface disposed thereon, a second reflective surface disposed on the housing distally of the first reflective surface and in axial alignment therewith, a first light conductive fiber in light coupled communication with the first reflective surface and a second light conductive fiber in light coupled communication with the second reflective surface, the first light conductive fiber in communication being in communication with a controlled analytical-light generating source and the second light conductive fiber being in communication with a light-collection analysis device. A generally curvilinear cover may be arranged to mate over a distal portion of the housing to enclose the reflective surfaces, the cover having at least one opening on an annular surface thereof to permit light delivery to the body tissue, and to permit light collection therethrough upon reflection from the body tissue. At least one of the reflective surfaces may comprise a mirrored member. Each of said light conductive fibers may have a distal end arranged within the housing, and the at least one of the light conductive fibers is in abutting relationship with a non-reflective surface of the mirrored member. At least one of the reflective surfaces may be disposed in a holding pocket. The reflective surface may be secured in the holding pocket by an adhesive. An index matching fluid may be disposed about the reflective surfaces to minimize back reflections thereto, from the outer sheath.

The invention may also comprise a catheter tip apparatus arranged in a catheter for the delivery and collection of an energy signal to permit subsequent computerized analysis of body tissue by the collected signal, comprising an optically transparent sheath enclosed elongated housing having a longitudinal axis of rotation. The housing may have a first reflective light delivery surface disposed thereon and a first reflective light collection surface disposed on the housing distally of the first reflective light delivery surface and in axial alignment therewith. A first light conductive fiber is in light coupled communication with the first reflective light delivery surface and a second light conductive fiber is in light coupled communication with the first reflective light collection surface, the first light conductive fiber in being in communication with a controlled analytical-light generating source and the second light conductive fiber being in communication with a light-collection analysis device, and a second reflective light collection surface may be disposed on the housing distally of the first reflective light collection, the second reflective collection surface may also be in communication with the controlled analytical-light generating source and in axial alignment therewith. The first and second reflective surfaces are thus arranged to permit deep tissue light energy penetration and collection and analysis thereby. Reflectors may otherwise be known as beam redirecting members comprised of aspheric members, planar, spherical, convex surfaces, concave surfaces, comprised of mirrors, dielectric mirrors, refractive index interfaces or diffractive optical elements.

The invention may also include a method of delivering and collecting a tissue-striking light energy signal from a first light fiber and adjacent delivery reflector and returning said light energy signal to a collection reflector adjacent a second light fiber for analysis and tissue treatment except when performed on a living body. The method includes spacing the collection reflector distally of the delivery reflector in a sheath enclosed elongated catheter housing tip, the housing having a longitudinal axis; disposing the reflectors at an angle with respect to the longitudinal axis of the elongated housing proportional to a numerical aperture of the first and second energy fibers. The method may include bathing the reflectors in an index matching fluid to minimize back reflection in the sheath enclosed housing, and directing the delivery light energy signal and the collection light energy signal so as to yield adjacent edges thereof that are parallel.

Thus what has been shown as a unique arrangement of structures for supporting energy carrying fibers such as flexible optical fibers to permit particular radiation to be delivered from one reflective surface and collected on a second adjacent reflective surface and analyzed in a computer apparatus at the proximal end of those fibers.

### Brief Description of the Drawings

The objects and advantages of the present invention will become more apparent when viewed in conjunction with the following drawings in which:
Figure 1 is an exploded view of a probe housing and an optical fiber arrangement adaptable for insertion into a mammalian body;
Figure 2 is a view similar to Figure I, showing the fiber components arranged within the elongated housing;
Figure 3 is an exploded view in perspective, of a prism and support frame arrangement and a housing for that support frame, for attachment on the distal end of a set of optical fibers;
Figure 4a is a view of a collection and delivery probe arranged on the distal end of a transmission coil in a perspective view thereof;
Figure 4b is an exploded view of the coil and probe components shown in Figure 4a;
Figure 5a is a perspective view of an assembly of an elongated housing having a pair of reflective surfaces arranged therewithin;
Figure 5b is a view similar to Figure 5a showing the frame components thereof without the surrounding elongated housing;
Figure 6a is a perspective view of a probe assembly showing a one piece housing arrangement with optical fibers in a counterwound coil;
Figures 6b, 6c and 6d are side elevational views of the housing shown in Figure 6a;
Figure 7 shows a perspective view of a fiber supporting catheter tip housing arrangement manufactured by additive and subtractive methods;
Figure 8 shows a schematic representation of multiple collection fibers in a housing; and
Figures 9a and 9b show reflectors arranged in their elongated housing for parallel light reflective beams.

### Detailed Description of the Preferred Embodiments

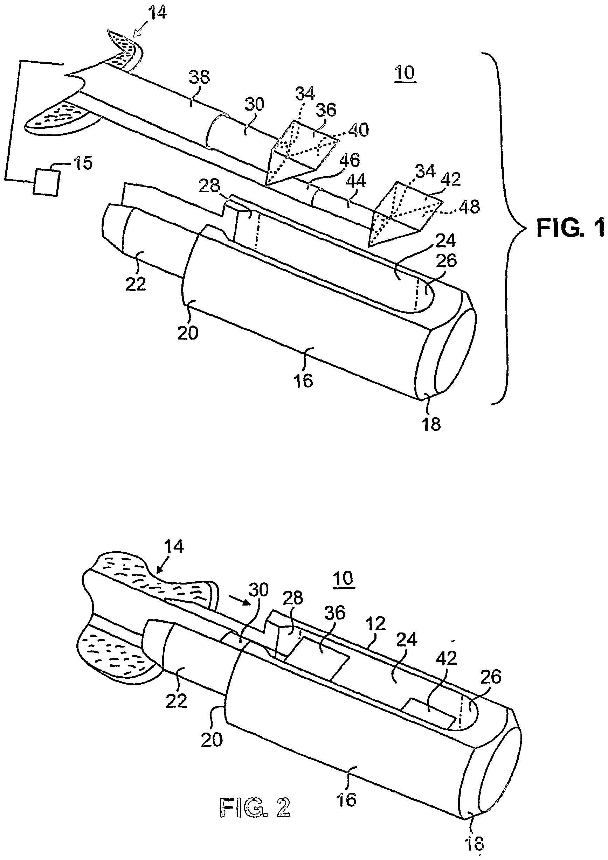

Referring now to the drawings in detail, and particularly to figure 1, there is shown a first embodiment of the present invention which comprises a catheter tip apparatus 10 and a method of use of that apparatus 10 except when performed on a living body to provide an analysis of body tissue using an energy spectrum analysis distributed and received by an elongated probe 12 introducable through a catheter sheath 14 into that body tissue. That introduction of the catheter sheath 14 and body probe 12 may be done through an endoscope, or other catheter-like devices, not shown, for such energy diagnosis and treatment of tissue by a proper computer apparatus 15. The energy analysis and treatment night include fluorescence spectroscopy, near infrared (NIR) reflectance spectroscopy, Raman spectroscopy, and optical coherence tomography, photodynamic drug activation, photonic ablation and thermal treatments.

The probe 12 of the present invention comprises an elongated, generally cylindrically shaped housing 16, as shown in figures 1 and 2, having a first or distal end 18 and a second or proximal end 20. The proximal end 20 has a stem 22 thereon of reduced diameter from the diameter of the distalmost 18 portion thereof. An elongated groove 24 is arranged to extend from the proximal end 20 of the stem 22 through towards the distal end 18 of the housing 16, as may be seen in figures 1 and 2. The groove 24 extends only through one side of the housing, and has an arrangement of angled shoulders 26 and 28 therein for providing snug receipt of the collector fiber 30 and the delivery fiber 32.

The collector fiber arrangement in this particular embodiment includes the elongated flexible collection fiber 30 having a distal end 34 to which a reflector or reflective surface 36 (i.e. mirrored/polished member) is attached. The collection fiber 30 has an outer buffer 38, for protection of the fiber and to minimize stray radiation therefrom. The reflector 36 has an angularly disposed reflective surface 40 thereon.

The delivery reflector 42 is attached to an optical delivery fiber 44 which is enclosed similarly by an outer buffer 46 such as a sheath for protection of the fiber and for minimization of light leakage. The delivery reflector 42 has an angled reflective surface 48 thereon. The collection fiber 30 and attached reflector 36 and the delivery fiber 44 and its attached reflector 42 jointly mate within the elongated receiving groove 24 within the stem and tip housing 16. The elongated groove 24 is preferably shaped to effect accurate positioning of the respective reflectors 36 and 42 therein, so as to emit radiation from the delivery reflector 42 and receive radiation reflected back from a body tissue sample through the collection (reflector) 36. Once the collection and delivery fibers 30 and 44 are within the housing 16, those joint fibers 30 and 44 may be inserted within the elongated catheter shaft 14 or rotatable coil as will be described hereinbelow.

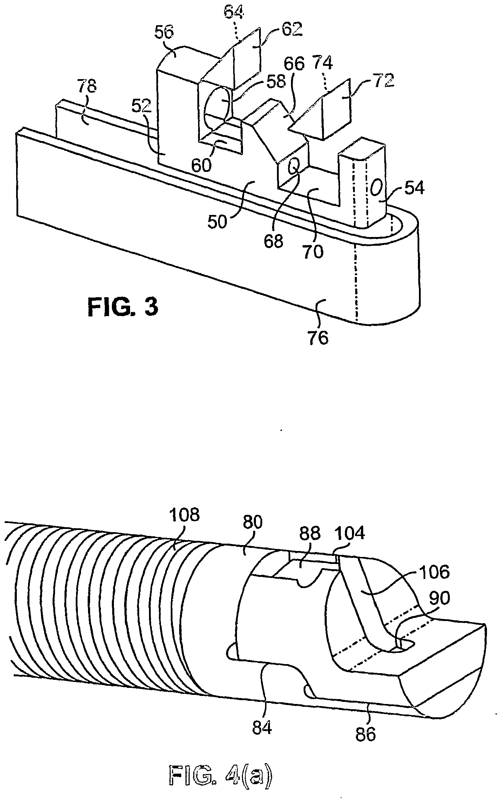

A further embodiment of the present invention shown in figure 3 and is disclosed as an elongated support frame 50 having a proximal end 52 and a distal end 54. The proximal end 52 includes an upstanding portion 56 through which a collection fiber support and alignment channel 58 is arranged (i.e. molded, drilled, machined). A rectilinearly-shaped holding pocket 60 is arranged distal of the first upstanding member 56 and is arranged to receive a reflective mirrored surface 62, (for example, a reflective collection member) having a reflecting surface 64 thereon. An upstanding midblock potion 66 is arranged centrally of the support frame 50 and has a delivery fiber support and alignment channel 68 arranged therewithin. The delivery fiber channel 68 extends parallel and adjacent the collection fiber channel 58. A second holding or receiving pocket 70 is similarly arranged distally adjacent the midblock portion 66 for adhesive receipt of a second reflector or mirrored surface (i.e. mirror or reflective member) 72 having a mirrored surface 74 thereon. The holding pockets 60 and 70 are constructed so as to accurately receive and align the respective first and second reflector arrangements (i.e. mirrors or reflective members) 62 and 72 to the desired angle for the desired photon delivery and photon collection from a target body tissue, not shown for clarity. The elongated support frame 50 is arranged within an elongated generally U-shaped housing 76, having an elongated channel 78 for receipt thereof. A delivery fiber and a collection fiber would be inserted within their respective channels 68 and 58 and the respective reflectors (i.e. reflective members/mirrors) 72 and 62 would be secured (i.e. affixed by adhesive) within those respective holding pockets 70 and 60.

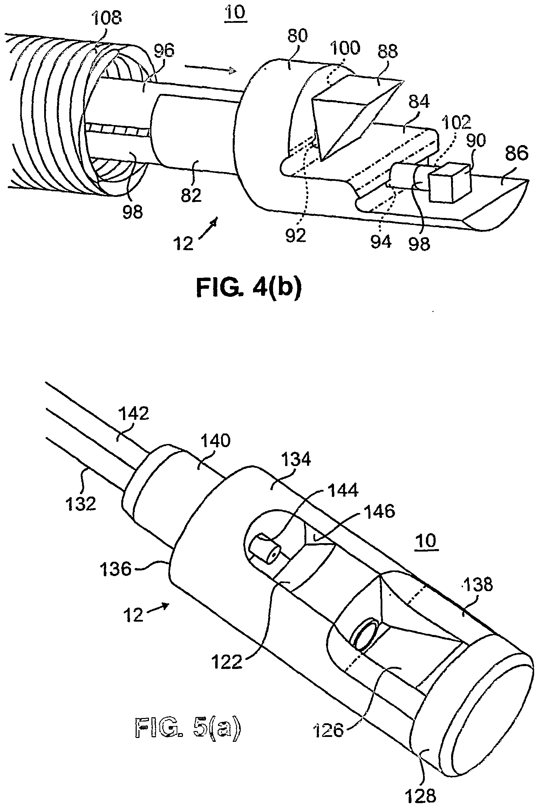

A further embodiment of the optical probe arrangement of the present invention is shown in figures 4(a) and 4 (b) and is characterized by a generally cylindrically shaped frame member 80 having a stepped down stem portion 82 on its proximal end thereof, as may be seen in figure 4(b). The frame member 80 is arranged so as to define a series of distal step portions 84 and 86, the first portion 84 of which is arranged to receive a collection reflector (i.e. reflective member or reflective surface) 88 and the second stepped portion 86 receives a delivery reflector (i.e. reflective member or reflective surface) 90. A bore or channel 92 and 94 is arranged through the frame 80 for servicing each particular reflector 88 and 90. Each respective channel 92 and 94 receives a collection fiber 96 or an optical fiber 98 which is arranged to abut a planar surface 100 and 102 of its respective reflector 88 and 90. A distal cover 104 as shown in figure 4(a) may be arranged so as to mate over the respective collection reflector 88 and delivery reflector 90 while having a slot 106 for passage of a delivered or received photonic signal therethrough. The delivery reflector 90 is thus permitted to emit photonic radiation and the collection prism 88 is permitted to receive photonic radiation when the frame 80 is assembled with the cover 104 and the stem 82 is attached and holding the particular fibers 96 and 98, and those fibers 96 and 98 are inserted within a hollow, torqueable transmission shaft such as a counter wound multifilar drive shaft coil arrangement 108 proximal of that frame member 80 and secured to the stem 82.

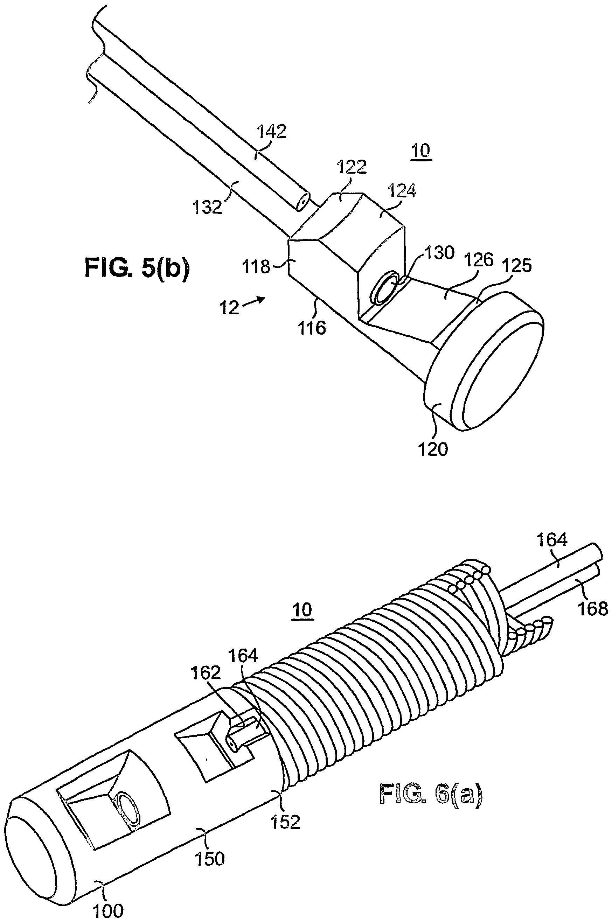

A further embodiment of the probe arrangement of the present invention is shown in figures 5(a) and 5(b) by an elongated frame member 116 having a proximal end 118 and a distal end 120, as may be best seen in
figure 5(b). The proximal end 118 of that frame member 116 is arranged so as to define an first angled reflective surface 122 at an upright portion 124 thereof, the distal end 120 of that frame member 116 having a second upright portion 125 with a second reflective surface 126 angularly disposed thereon with an oval end cap 128 thereon. A bore 130 is arranged through the upright portion 124 on the proximal end 118 of the elongated frame member 116 so as to define a receiving channel for a delivery fiber 132 to be inserted therewithin. The entire elongated frame 116 is inserted into an elongated housing 134, as shown in figure 5(a), having a proximal end 136 and a distal end 138. The proximal end 136 of the housing 134 has a stem 140 with a stepped down diameter which encloses the delivery fiber 132 and a collection fiber 142. The stem 140 of the housing 134 is arranged to receive the collection fiber 142 which is arranged to receive reflective signals from its reflection surface 122 arranged onto the upstanding portion 124 of the elongated frame member 116. The housing 134 has an opening 144 arranged longitudinally therein, as shown in figure 5(a), so as to receive the elongated frame member 116. The housing 134 also has a slot 146 cut through its side portion thereof so as to permit the photonic energy signal to be delivered and received by the respective reflective surfaces 126 and 122 contained therewithin. The end cap 128 on the distalmost end 120 of the elongated frame member 116 abuts the distalmost end 138 of the elongated housing 134 to define a smooth continuous surface therearound.

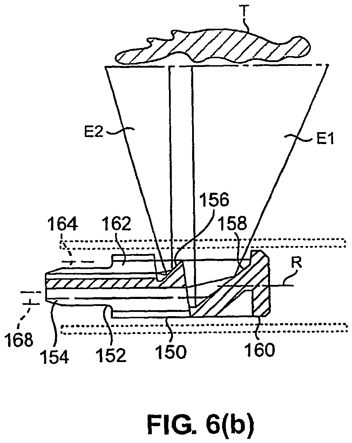

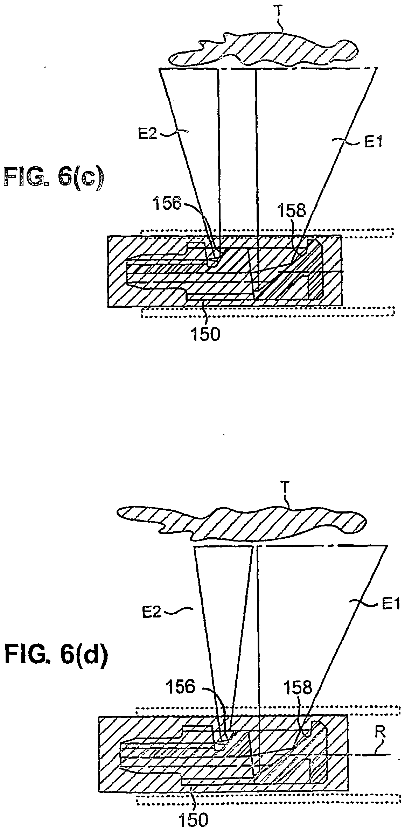

A further embodiment of the present invention resides in a one piece housing 150 of generally cylindrical shape, (as may be seen in figures 6(a), 6(b), 6(c) and 6(d)), having a proximal portion 152 of stepped down diameer defining a reduced-diameter fiber-enclosing stem 154. A first reflective collection surface 156 is disposed at a particularly desired angle near the stem 154 of the housing 150, and a second reflective surface 158 is disposed onto the elongated housing 150 adjacent its distalmost end 160. A first channel or bore 162 is arranged through the proximal end 152 of the housing 150 adjacent the stem 154 so as to snugly and alignably receive a signal collection fiber 164. A second channel or bore 166 is also arranged through the proximal half of the elongated housing 150 so as to alignably support a delivery fiber 168 therethrough. The stem 154 may be received in a pair of hollow, flexible, counterwound coils 170, as shown in figure 6(a), which coils 170 that function as a torqueable transmission shaft, is secured to the stem 154 and surrounds the delivery and collection fibers 168 and 164 received therethrough. The first and second reflective surfaces 156 and 158 arranged into the housing 150, in this embodiment, and which is depicted by light energy waves E1 and E2 reflecting with respect thereto, may be skewed (non parallel or curvilinear) with respect to one another so as to emit and receive signals from any particular direction with respect to the surface of an adjacent reflective tissue "T". Figure 6(c) represents the housing 150 with the respective reflective surfaces 156 and 158 being spaced further longitudinally apart than is depicted in figure 6(b). This permits different scattering patterns E1 and E2 to be delivered and hence received by the catheter tip apparatus 10. Figure 6(d) represents the manufactured reflective surfaces 156 and 158 at a skewed angle with respect to one another to present further signal delivery and collection characteristics with respect thereto. The fiber bores 154 and 162 are oppositely aligned and diametrically disposed across the longitudinal axis of rotation "R" of the housing 150. This minimizes eccentric rotation of the housing 150, the fibers borne therein and signal distortion during rotation of the housing 150 in a body vessel during analysis of tissue "T".

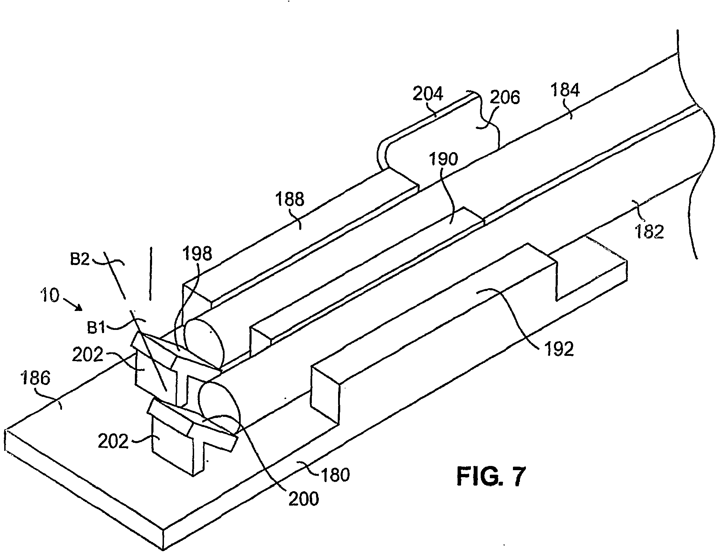

A yet further embodiment of the present invention is shown in figure 7, wherein a platform 180 relates to a method of constructing a catheter tip arrangement 10 for support of a plurality of two or more optical delivery and collection fibers 182 and 184, which "support" construction permits minimization of component size and adaptive angularity of reflection of the delivery and collection beams B1 and B2; Such a support platform 180 may be accomplished by micro-machining construction where additive or subtractive processing such as for example: etching, plating, sputtering, vapor deposition and subtractive processing such as etching, laser cutting and ablation permits finite adjustment to the dimensions. The support platform 180 comprises a base 186 upon which an arrangement of elongated, parallel bosses 188, 190 and 192 are "grown", the bosses 188, 190 and 192 defining between them, a pair of parallel slots 194 and 196 into which a delivery and a collection fiber 182 and 184 may be respectively mated. A mirror surface 198 and 200 and support struts 200 and 202 are spaced at the distalmost location of the fibers 182 and 184, which mirror surfaces 198 and 200 may be curved or manipulably bent to the desired angle for maximizing optical analysis and tissue treatment thereby. This embodiment shown in figure 7 contemplates the use of index-matching fluids 206 added to any gap between a catheter sheath 204 surrounding the fibers 182 and 184, to reduce any back reflections from the interior of the protective sheath/transmission window.

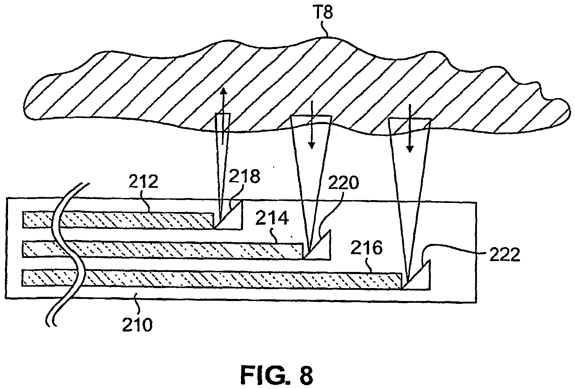

Figure 8 shows a schematic representation of a catheter tip disposed elongated housing 210 having an optical energy delivery fiber 212 and a first optical energy collection fiber 214 and a second optical energy collection fiber 216 in optical communication with a mammalian tissue "T8". Each fiber 212, 214 and 216 have a reflective surface 218, 220 and 222 disposed distally thereof respectively, as shown in figure 8. The reflective surfaces 218, 220 and 222 are axially spaced apart from one another. Multiple collection fibers 214 and 216 with axially spaced apart collection reflective surfaces 220 and 222 permit collection and analysis of light that has penetrated more deeply into the tissue "T8". The reflective surfaces 218, 220 and 222 may be aspheric volumes, or flat, convex, concave or curved members having an arcuate surface to present a straight reflective beam, a spread-out beam, a focused beam which may overlap one another, be parallel to one another, or in alignment with one another. Multiple collectors 220 and 222 permits the receiving or collection of light emitted from a single source but collected from more than one collector arranged at spaced apart locations within the tissue being investigated. The beams may have a delivery numerical aperture NA of between NA=0.1 and NA =0.6 and a collection numerical aperture NA of between NA=0.1 and NA = 0.7.

Bean redirecting members such as mirrors preferably have separations of about 0.1 mm and 2mm.

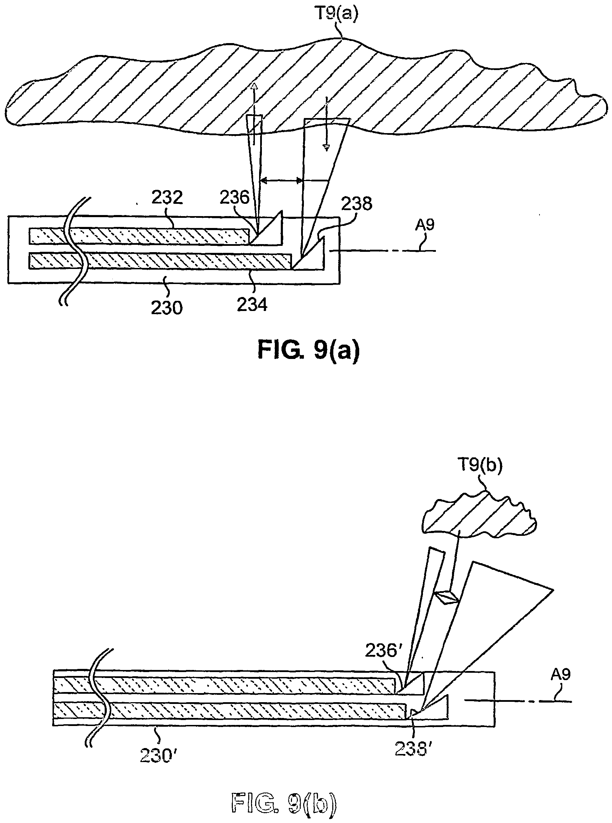

A more preferential delivery and collection beam geometry is shown in figures 9a and 9b having a catheter tip disposed elongated housing 230 having an optical delivery fiber 232 and an optical energy collection fiber 234 in optical communication with mammalian tissue "9a". A reflective surface 236 delivers a generally radially delivered light beam L9 and a reflective surface 238 collects the generally radially directed returning light beam L9. Adjacent portions of the delivered beam and the returning beam in this embodiment are parallel, because the delivery and collection reflectors 236 and 238 are disposed at chosen angles proportional to the numerical aperture of the delivery and collection fibers 232 and 234 to yield energy beam having edges that are parallel to permit distance independent delivery-collector separation, such angularity of the reflectors 236' and 238' being shown at a less steep angle with respect to the longitudinal axis A9, in an elongated housing 230', represented in figures 9b. It is also contemplated that each fiber may be utilized for both delivery and collection of light energy.

Thus what has been shown as a unique arrangement of structures for supporting energy carrying fibers or waveguide elements to permit particular radiation to be delivered from at least one beam redirecting member such as a reflective surface and collected on the same or at least a second adjacent beam redirecting member such as a second reflective surface and analyzed in a light signal analyzer computer apparatus connected to the proximal end of those fibers, waveguides or beam bearing members.

## Claims

1. A catheter tip apparatus arranged in a catheter for the delivery and collection of an energy signal to permit subsequent light energy beam analysis of body tissue by the collected signal, comprising:
an elongated housing (16,50,80,116,126,158,180,200) supporting a first reflective surface (42,74,90,126,158,200) for emitting radiation in a first beam toward a target body tissue and a second reflective surface (36,64,88,122,156,198) for receiving radiation reflected back from the target body tissue, said first reflective surface (42,74,90,126,158,200) and said second reflective surface (36,64,88,122,156,198) being longitudinally spaced apart from one another and aligned to an angle for photon delivery and collection from the target body tissue;
a first flexible, elongated energy bearing delivery fiber (44,98,132,168,182) having a distalmost end arranged adjacent said first reflective surface;
a second flexible, elongated energy bearing collection fiber (30,96,142,164,184) having a distalmost end arranged adjacent said second reflective surface; and **characterised in that** said housing is rotatably supported within a flexible catheter sheath (14) for insertion of said catheter into a mammalian body for tissue analysis thereof.

2. The catheter tip apparatus as recited in claim 1, wherein said housing (16) comprises a frame member having a slot (24) arranged therein for receipt and alignment of said first and said second reflective surfaces (36,42).

3. The catheter tip apparatus as recited in claim 1, wherein said first reflective surface and said second reflective surface each comprise a beam redirecting member.

4. The catheter tip apparatus as recited in claim 2, wherein said slot (24) has shoulders (26,28) therein to guideably secure and accurately align said reflective surfaces (36,42) therein.

5. The catheter tip apparatus as recited in claim 2, wherein said housing (16) has a proximalmost stem portion (22) for receipt into a catheter sheath (14) to permit manipulation of said tip from a proximal location.

6. The catheter tip apparatus as recited in claim 1, wherein said housing comprises a frame member having a proximal end (52) and a distal end (54) with an upstanding proximal block (56) and an upstanding midblock (66) each block having a pocket (60,70) thereadjacent for receipt of a reflective surface (62,72) attachable therein.

7. The catheter tip apparatus as recited in claim 6, wherein said reflective surface comprises a mirror glued into said pocket.

8. The catheter tip apparatus as recited in claim 6, wherein each of said upstanding blocks (56,66) has a bore (58,68) therethrough for receipt of one of said energy bearing fibers.

9. The catheter tip apparatus as recited in claim 1, wherein said housing comprises an elongated generally cylindrically shaped frame member (80) with a proximal end and a distal end, said frame member having at least two steps (84,86) thereon of decreasing thickness in the distal direction, each of said steps having a reflective surface (88,90) mounted thereon, said proximal end having a stem portion (82) of reduced diameter, to permit rotative receipt within a catheter sheath.

10. The catheter tip apparatus as recited in claim 9, wherein said frame member has a cover (104) member arranged to mate over said steps (84,86) and said reflective surfaces.

11. The catheter tip apparatus as recited in claim 10, wherein said cover member has an axially arranged slot thereon through part of its longitudinal length, said slot being disposed radially adjacent each of said reflective surfaces to permit delivery and reflected collection of an energy beam therethrough.

12. The catheter tip apparatus as recited in claim 9, wherein said stem portion is secured to a multi-layered, elongated coil spring arrangement (108) to permit twisting control of said catheter tip within a mammalian body component.

13. The catheter tip apparatus as recited in claim 1, wherein said reflective surfaces are unitary portions of said housing.

14. The catheter tip apparatus as recited in claim 13 wherein said housing (116) has a proximal end (118) and a distal end (120), and said proximal end (118) mates with a housing enclosure, said enclosure (134) providing a securement means (140) for said energy collecting fiber (142) and said housing (134) provides a securement means (140) for said delivery fiber (132).

15. The catheter tip apparatus as recited in claim 14, wherein said housing enclosure (134) attached to said proximal end (118) of said housing (116) has a longitudinally directed elongated slot (144) therein, said slot (144) being in radial alignment with said reflective surfaces (122,126) formed on said housing (116) to permit transmission and collection of radiant energy via said respective reflective surfaces to a computerized analysis system.

16. The catheter tip apparatus as recited in claim 1, wherein said housing (150) comprises a cylindrically shaped member having said first and second reflective surfaces (156,158) machined thereon, and wherein said first and second reflective surfaces (156,158) are non-parallel with respect to one another.

17. The catheter tip apparatus as recited in claim 16, wherein said first and second fibers (164,168) are diametrically oppositely aligned with respect to one another about a longitudinal axis of rotation of said housing (150) to minimize eccentricity of rotation of said catheter housing during rotation of said housing (150) in a body tissue.

18. The catheter tip apparatus as recited in claim 1, wherein said housing includes a reflective surface (198,200) which is bendable to effect directional change of an energy beam reflecting therefrom.

19. The catheter tip apparatus as recited in claim 18, wherein said housing has accumulation components defining a fiber alignment slot (194,196) for miniaturization of said tip.

20. The catheter tip apparatus of claim 1, wherein said first flexible, elongated energy bearing delivery is fiber is in communication with a controlled analytical-light-generating source and said second flexible, elongated energy bearing collection fiber is in communication with a light-collection analysis device.

21. The catheter tip apparatus of claim 1 or 20,
wherein the first flexible, elongated energy bearing delivery fiber (44, 98,132, 168, 182) is a light conductive fiber in light-coupled communication with said first reflective surface (42, 74, 90); and
wherein said second flexible, elongated energy bearing collection fiber (30, 96, 142, 164, 184)is a light conductive fiber in light-coupled communication with said second reflective surface (36, 64, 88, 122, 156, 198).

22. The catheter tip apparatus as recited in claim 1 or 20, wherein said first reflective surface (42,74,126,158,200) is dimensionally larger than said second reflective surface (36,64,88,122,156,198).

23. The catheter tip apparatus as recited in claim 1 or 20, wherein said first reflective surface (218) is curvilinear.

24. The catheter tip apparatus as recited in claim 1 or 20, wherein said first reflective surface is non-parallel with respect to said second reflective surface.

25. The catheter tip apparatus as recited in claim 1 or 20, wherein at least one of said first and second reflective surfaces are spaced apart from said fibers.

26. The catheter tip apparatus as recited in claim 1 or 20, wherein said first relfective surface is disposed radially within and spaced from the perimeter of said housing to permit a spreading of a light beam from said first reflective surface onto said body tissue.

27. The catheter tip apparatus as recited in claim 25, having an index matching fluid (206) arranged between a distal end of said fiber (182,184) and said reflective surface (198,200).

28. The catheter tip apparatus as recited in claim 25, wherein said reflective surface is positioned in a holding pocket (60) arranged in said housing.

29. The catheter tip apparatus as recited in claim 28, wherein said reflective surface comprises a mirrored member.

30. The catheter tip apparatus as recited in claim 28, wherein said holding pocket (60) is utilized to align said reflective surface with respect to said housing (50).

31. The catheter tip apparatus as recited in claim 1 or 20, wherein said conductive light fibers are each individually arranged within a bore (58,68,92,94,130,162,166) disposed within said housing.

32. The catheter tip apparatus as recited in claim 1 or 20, wherein said fibers are equally diametrically opposed about an axis of rotation of said housing to provide balance thereto and minimize eccentricity during rotation thereof.

33. The catheter tip apparatus as recited in claim 1 or 20, wherein said first reflective surface (236) and said second reflective surface (238) are disposed at an angle proportional to the numerical aperture of said first and second fibers (232,234), to yield a light beam with adjacent edges that are parallel to one another, to permit a distance independent delivery reflector-collector reflector separation.

34. A method of delivering and collecting a tissue-striking light energy signal from a first light bearing member and adjacent delivery beam redirecting member for emitting the light energy signal toward a target body tissue, except when performed on a living being, and returning said light energy signal from the target body tissue to a collection beam redirecting member adjacent a second light bearing member for analysis and tissue treatment, in a light bearing arrangement, including: spacing said collection beam redirecting member distally of said delivery beam redirector member aligned to an angle for photon delivery and collection from the target body tissue in a flexible sheath enclosed elongated catheter housing tip for insertion into a mammalian body tissue for analysis thereof, wherein said housing is rotatably supported within the sheath, said housing having a longitudinal axis disposing said beam redirecting members at an angle with respect to said longitudinal axis of said elongated housing proportional to a numerical aperture of said first and second energy fibers.

35. The method as recited in claim 34, including: bathing said reflectors in an index matching fluid to minimize back reflection in said sheath enclosed housing.

36. The method as recited in claim 34, including: directing said delivery light energy signal and said collection light energy signal so as to yield adjacent edges thereof that are parallel.

37. The method as recited in claim 34, including: delivering and collecting light from common fibers in said light fiber bearing arrangement.

38. The method as recited in claim 34, therein said numerical apertures for each of said beam redirectors are different from one another.

39. The method as recited in claim 34, wherein said beam redirectors are reflectors.

40. The method as recited in claim 34, wherein said beam bearing members comprise optical fibers.

41. The method as recited in claim 34, wherein said beam bearing members comprise waveguides.

## Patentansprüche

1. Katheterspitzenvorrichtung, die in einem Katheter angeordnet ist, für die Zustellung und die Aufnahme eines Energiesignals, um eine folgende Lichtenergiestrahlanalyse von Körpergewebe durch das aufgenommene Signal zu ermöglichen, wobei die Vorrichtung folgendes umfasst:
ein elongiertes Gehäuse (16, 50, 80, 116, 126, 158, 180, 200), welches eine reflektierende Oberfläche (42, 74, 90, 126, 158, 200) zur Emission von Strahlung in einem ersten Strahl in Richtung eines Zielkörpergewebes trägt, und mit einer zweiten reflektierenden Oberfläche (36, 64, 88, 122, 156, 198) zum Empfang von Strahlung, die von dem Zielkörpergewebe zurückgestrahlt wird, wobei die genannte erste reflektierende Oberfläche (42, 74, 90, 126, 158, 200) und die genannte zweite reflektierende Oberfläche (36, 64, 88, 122, 156, 198) longitudinal zueinander mit Zwischenabständen angeordnet und in einem Winkel für die Photonenzustellung und -aufnahme von dem Zielkörpergewebe ausgerichtet sind;
eine erste flexible, elongierte, Energie führende Zustellungsfaser (44, 98, 132, 168, 182) mit einem entferntesten Ende, das angrenzend an der genannten ersten reflektierenden Oberfläche angeordnet ist;
eine zweite flexible, elongierte, Energie führende Aufnahmefaser (30, 96, 142, 164, 184) mi einem entferntesten Ende, das angrenzend an die genannte zweite reflektierende Oberfläche angeordnet ist; und **dadurch gekennzeichnet, dass** das genannte Gehäuse drehbar in einer flexiblen Kathetherhülle (14) getragen wird für eine Einführung des genannten Katheters in einen Säugetierkörper für eine Gewebeanalyse des Körpers.

2. Katheterspitzenvorrichtung nach Anspruch 1, wobei das genannte Gehäuse (16) ein Rahmenelement umfasst, das einen Schlitz (24) aufweist, der darin zur Aufnahme und Ausrichtung der genannten ersten und zweiten reflektierenden Oberflächen (36, 42) angeordnet ist.

3. Katheterspitzenvorrichtung nach Anspruch 1, wobei die genannte erste reflektierende Oberfläche und die genannte zweite reflektierende Oberfläche jeweils ein Strahlumrichtungselement umfassen.

4. Katheterspitzenvorrichtung nach Anspruch 2, wobei der genannte Schlitz (24) darin Schultern (26, 28) aufweist, um die genannten reflektierenden Oberflächen (36, 42) darin führend zu sichern und präzise auszurichten.

5. Katheterspitzenvorrichtung nach Anspruch 2, wobei das genannte Gehäuse (16) ein nächstes Schaftteilstück (22) zur Aufnahme in einer Katheterhülle (154) aufweist, um eine Manipulation der genannten Spitze von einer proximalen Position zu ermöglichen.

6. Katheterspitzenvorrichtung nach Anspruch 1, wobei das genannte Gehäuse ein Rahmenelement umfasst, mit einem proximalen Ende (52) und einem distalen Ende (54) mit einem aufrecht stehenden proximalen Block (56) und einem aufrecht stehenden mittleren Block (66), wobei jeder block eine benachbarte Tasche (60, 70) zur Aufnahme einer darin anbringbaren reflektierenden Oberfläche (62, 72) aufweist.

7. Katheterspitzenvorrichtung nach Anspruch 6, wobei die genannte reflektierende Oberfläche einen in die genannte Tasche geklebten Spiegel umfasst.

8. Katheterspitzenvorrichtung nach Anspruch 6, wobei jeder der genannten aufrecht stehenden Blöcke (56, 66) eine dort hindurch gehenden Bohrung (58, 68) zur Aufnahme einer der Energie führenden Fasern aufweist.

9. Katheterspitzenvorrichtung nach Anspruch 1, wobei das genannte Gehäuse ein elongiertes, allgemein zylinderförmiges Rahmenelement (80) mit einem proximalen Ende und einem distalen Ende umfasst, wobei das genannte Rahmenelement daran mindestens zwei Stufen (84, 86) mit abnehmender Dicke in die distale Richtung aufweist, wobei jede der genannten Stufen eine daran angebrachte reflektierende Oberfläche (88, 90) aufweist, wobei das genannte proximale Ende ein Schaftteilstück (82) mit reduziertem Durchmesser aufweist, um eine Aufnahme in einer Katheterhülle durch Rotation zu ermöglichen.

10. Katheterspitzenvorrichtung nach Anspruch 9, wobei das genannte Rahmenelement ein Abdeckungselement (104) aufweist, das so angeordnet ist, dass es über den genannten Stufen (84, 86) und den genannten reflektierenden Oberflächen zusammenpasst.

11. Katheterspitzenvorrichtung nach Anspruch 10, wobei das genannte Abdeckungselement daran einen axial angeordneten Schlitz durch einen Teil dessen Längslänge aufweist, wobei der genannte Schlitz radial angrenzend an die genannten reflektierenden Oberflächen angeordnet ist, um die Zustellung und reflektierte Aufnahme eines Energiestrahls dort hindurch zu ermöglichen.

12. Katheterspitzenvorrichtung nach Anspruch 9, wobei das genannte Schaftteilstück an einer mehrschichtigen, elongierten Schraubenfederanordnung (108) gesichert ist, um eine Drehsteuerung der genannten Katheterspitze in einer Säugetierkörperkomponente zu ermöglichen.

13. Katheterspitzenvorrichtung nach Anspruch 1, wobei die genannten reflektierenden Oberflächen unitäre Teilstücke des genannten Gehäuses darstellen.

14. Katheterspitzenvorrichtung nach Anspruch 13, wobei das genannte Gehäuse (116) ein proximales Ende (118) und ein distales Ende (120) aufweist, und wobei das genannte proximale Ende (118) mit einer Gehäuseeinfassung zusammenpasst, wobei die genannte Einfassung (134) eine Befestigungseinrichtung (140) für die genannte Energie führende Faser (142) bereitstellt, und wobei das genannte Gehäuse (134) eine Befestigungseinrichtung (140) für die genannte Zustellungsfaser (132) bereitstellt.

15. Katheterspitzenvorrichtung nach Anspruch 14, wobei die genannte Gehäuseeinfassung (134), die an dem genannten proximalen Ende (118) des genannten Gehäuses (116) angebracht ist, darin eine längs ausgerichteten elongierten Schlitz (144) aufweist, wobei sich der genannte Schlitz (144) in radialer Ausrichtung mit den genannten reflektierenden Oberflächen (122, 126) befindet, die an dem genannten Gehäuse (116) ausgebildet sind, um die Übermittlung und Aufnahme von Strahlungsenergie über entsprechende reflektierende Oberflächen an ein rechnergestütztes Analysesystem zu ermöglichen.

16. Katheterspitzenvorrichtung nach Anspruch 1, wobei das genannte Gehäuse (150) ein zylinderförmiges Element umfasst, das daran gearbeitete erste und zweite reflektierende Oberflächen (156, 158) aufweist, und wobei die genannten ersten und zweiten reflektierenden Oberflächen (156, 158) im Verhältnis zueinander nicht parallel sind.

17. Katheterspitzenvorrichtung nach Anspruch 16, wobei die genannten ersten und zweiten Fasern (164, 158) genau entgegengesetzt zueinander ausgerichtet sind um eine longitudinale Rotationsachse des genannten Gehäuses (150), um die Exzentrizität der Rotation des genannten Kathetergehäuses während der Rotation des genannten Gehäuses (150) in einem Körpergewebe zu minimieren.

18. Katheterspitzenvorrichtung nach Anspruch 1, wobei das genannte Gehäuse eine reflektierende Oberfläche (198, 200) aufweist, die biegbar ist, um eine Richtungsänderung eines davon reflektierten Energiestrahls zu bewirken.

19. Katheterspitzenvorrichtung nach Anspruch 18, wobei das genannte Gehäuse Akkumulationskomponenten aufweist, welche einen Faserausrichtungsschlitz (194, 196) zur Miniaturisierung der genannten Spitze zu definieren.

20. Katheterspitzenvorrichtung nach Anspruch 1, wobei sich die genannte erste flexible, elongierte, Energie führende Zustellungsfaser in Übertragungsverbindung mit einer gesteuerten analytischen, Licht erzeugenden Quelle befindet, und wobei sich die genannte zweite flexible, elongierte, Energie führende Aufnahmefaser in Übertragungsverbindung mit einer Lichtaufnahme-Analysevorrichtung befindet.

21. Katheterspitzenvorrichtung nach Anspruch 1 oder 20,
wobei es sich bei der ersten flexiblen, elongierten, Energie führenden Zustellungsfaser (44, 98, 132, 168, 192) um eine Licht leitende Faser in lichtgekoppelter Übertragungsverbindung mit der genannten ersten reflektierenden Oberfläche (42, 74, 90) handelt; und
wobei es sich bei der genannten zweiten flexiblen, elongierten, Energie führenden Aufnahmefaser (30, 96, 142, 164, 184) um eine Licht leitende Faser in lichtgekoppelter Übertragungsverbindung mit der genannten zweiten reflektierenden Oberfläche (36, 64, 88, 122, 156, 198) handelt.

22. Katheterspitzenvorrichtung nach Anspruch 1 oder 20, wobei die genannte erste reflektierende Oberfläche (42, 74, 126, 158, 200) eine größere Dimension aufweist als die genannte zweite reflektierende Oberfläche (36, 64, 88, 122, 156, 198).

23. Katheterspitzenvorrichtung nach Anspruch 1 oder 20, wobei die genannte erste reflektierende Oberfläche (218) krummlinig ist.

24. Katheterspitzenvorrichtung nach Anspruch 1 oder 20, wobei die genannte erste reflektierende Oberfläche im Verhältnis zu der genannten zweiten reflektierenden Oberfläche nicht parallel ist.

25. Katheterspitzenvorrichtung nach Anspruch 1 oder 20, wobei mindestens eine der genannten ersten und zweiten reflektierenden Oberflächen von den genannten Fasern räumlich getrennt angeordnet ist.

26. Katheterspitzenvorrichtung nach Anspruch 1 oder 20, wobei die genannte erste reflektierende Oberfläche radial innerhalb und mit Zwischenabstand zu dem Perimeter des genannten Gehäuses angeordnet ist, um eine Ausbreitung eines Lichtstrahls von der genannten ersten reflektierenden Oberfläche auf das genannte Körpergewebe zu ermöglichen.

27. Katheterspitzenvorrichtung nach Anspruch 25, mit einem Index-Matching-Fluid (206), das zwischen einem distalen Ende der genannten Faser (182, 184) und der genannten reflektierenden Oberfläche (198, 200) angeordnet ist.

28. Katheterspitzenvorrichtung nach Anspruch 25, wobei die genannte reflektierende Oberfläche in einer Haltetasche (60) positioniert ist, die in dem genannten Gehäuse angeordnet ist.

29. Katheterspitzenvorrichtung nach Anspruch 28, wobei die genannte reflektierende Oberfläche ein gespiegeltes Element umfasst.

30. Katheterspitzenvorrichtung nach Anspruch 28, wobei die genannte Haltetasche (60) eingesetzt wird, um die genannte reflektierende Oberfläche in Bezug auf das genannte Gehäuse (50) auszurichten.

31. Katheterspitzenvorrichtung nach Anspruch 1 oder 20, wobei die genannten leitfähigen Lichtfasern jeweils einzeln in einer Bohrung (58, 69, 92, 94, 130, 162, 166) angeordnet sind, die sich in dem genannten Gehäuse befinden.

32. Katheterspitzenvorrichtung nach Anspruch 1 oder 20, wobei die genannten Fasern übereinstimmend genau entgegengesetzt um eine Rotationsachse des genannten Gehäuses angeordnet sind, um diesbezüglich ein Gleichgewicht bereitzustellen und um die Exzentrizität während der Rotation der Achse zu minimieren.

33. Katheterspitzenvorrichtung nach Anspruch 1 oder 20, wobei die genannte erste reflektierende Oberfläche (236) und die genannte zweite reflektierende Oberfläche (238) in einem Winkel angeordnet sind, der proportional ist zu der numerischen Apertur der genannten ersten und zweiten Fasern (232, 234), um einen Lichtstrahl mit angrenzenden Rändern zu ergeben, die parallel zueinander sind, um eine abstandsunabhängige Separation zwischen Zustellungsreflektor und Aufnahmereflektor zu ermöglichen.

34. Verfahren für die Zustellung und die Aufnahme eines auf Gewebe treffenden Lichtenergiesignals von einem ersten Licht führenden Element, und mit einem benachbarten Zustellungsstrom-Umleitungselement zum Emittieren des Lichtenergiesignals in Richtung eines Zielkörpergewebes, ausgenommen dann, wenn eine Ausführung an einem Lebewesen erfolgt, und wobei das genannte Lichtenergiesignal von dem Zielkörpergewebe zu einem Aufnahmestrahl-Umleitungselement angrenzend an ein zweites Licht führendes Element zur Analyse und Gewebebehandlung zurückgeführt wird in einer Licht führenden Anordnung, wobei das Verfahren folgendes aufweist: die Abstandsanordnung des genannten Aufnahmestrahl-Umleitungselements distal zu dem genannten Zustellungsstrahl-Umleitungselement, ausgerichtet in einem Winkel für die Photonenzustellung und -aufnahme von dem Zielkörpergewebe in einer elongierten Kathetergehäusespitze, die in einer flexiblen Hülle eingeschlossen ist, zum Einführen in ein Säugetierkörpergewebe zu dessen Analyse, wobei das genannte Gehäuse drehbar innerhalb der Hülle getragen wird, wobei das genannte Gehäuse eine Längsachse aufweist, welche die genannten Strahlumleitungselemente in einem Winkel im Verhältnis zu der genannten Längsachse des genannten elongierten Gehäuses proportional zu einer numerischen Apertur der genannten ersten und zweiten Energiefasem anordnet.

35. Verfahren nach Anspruch 34, wobei dieses folgendes aufweist: das Baden der genannten Reflektoren in einem Index-Matching-Fluid zum Minimieren der Rückstrahlung in dem genannten von einer Hülle eingeschlossenen Gehäuse.

36. Verfahren nach Anspruch 34, wobei dieses folgendes aufweist: das Richten des genannten Zustellungs-Lichtenergiesignals und des genannten Aufnahme-Lichtenergiesignals, so dass benachbarte Ränder dieser resultieren, die parallel sind.

37. Verfahren nach Anspruch 34, wobei dieses folgendes aufweist: das Zustellen und Aufnehmen von Licht von gemeinsamen Fasern in der genannten Licht führenden Anordnung.

38. Verfahren nach Anspruch 34, wobei sich darin die genannten numerischen Aperturen für jede der genannten Strahlumleitungseinrichtungen voneinander unterscheiden.

39. Verfahren nach Anspruch 34, wobei es sich bei den genannten Strahlumleitungseinrichtungen um Reflektoren handelt.

40. Verfahren nach Anspruch 34, wobei die genannten Strahl führenden Elemente Lichtwellenleiter umfassen.

41. Verfahren nach Anspruch 34, wobei die genannten Strahl führenden Elemente Wellenleiter umfassen.

## Revendications

1. Appareil formant pointe de cathéter agencé dans un cathéter pour l'application et la collecte d'un signal d'énergie afin de permettre une analyse subséquente de faisceau d'énergie lumineuse de tissu corporel par le signal collecté, comprenant :
un boîtier allongé (16, 50, 80, 116, 126, 158, 180, 200) supportant une première surface réfléchissante (42, 74, 90, 126, 158, 200) pour émettre un rayonnement dans un premier faisceau vers un tissu corporel cible et une seconde surface réfléchissante (36, 64, 88, 122, 156, 198) pour recevoir un rayonnement réfléchi par le tissu corporel cible, ladite première surface réfléchissante (42, 74, 90, 126, 158, 200) et ladite seconde surface réfléchissante (36, 64, 88, 122, 156, 198) étant longitudinalement espacées l'une de l'autre et alignées à un angle pour une application et une collecte de photons à partir du tissu corporel cible ;
une première fibre d'application porteuse d'énergie allongée flexible (44, 98, 132, 168, 182) ayant une extrémité la plus distale agencée adjacente à ladite première surface réfléchissante ;
une seconde fibre de collecte porteuse d'énergie allongée flexible (30, 96, 142, 164, 184) ayant une extrémité la plus distale agencée adjacente à ladite seconde surface réfléchissante ; et **caractérisé en ce que** ledit boîtier est supporté de manière rotative à l'intérieur d'une gaine de cathéter souple (14) pour insertion dudit cathéter dans un corps de mammifère pour l'analyse de ses tissus.

2. Appareil formant pointe de cathéter selon la revendication 1, dans lequel ledit boîtier (16) comprend un élément formant cadre ayant une fente (24) agencée à l'intérieur pour recevoir et aligner lesdites première et seconde surfaces réfléchissantes (36, 42).

3. Appareil formant pointe de cathéter selon la revendication 1, dans lequel ladite première surface réfléchissante et ladite seconde surface réfléchissante comprennent chacune un élément de renvoi de faisceau.

4. Appareil formant pointe de cathéter selon la revendication 2, dans lequel ladite fente (24) a des épaulements (26, 28) à l'intérieur pour fixer avec guidage et aligner avec précision lesdites surfaces réfléchissantes (36, 42) à l'intérieur.

5. Appareil formant pointe de cathéter selon la revendication 2, dans lequel ledit boîtier (16) a une partie de tige la plus proximale (22) pour réception dans une gaine de cathéter (14) pour permettre la manipulation de ladite pointe à partir d'un emplacement proximal.

6. Appareil formant pointe de cathéter selon la revendication 1, dans lequel ledit boîtier comprend un élément formant cadre ayant une extrémité proximale (52) et une extrémité
distale (54) avec un bloc proximal vertical (56) et un bloc central vertical (66), chaque bloc ayant une poche (60, 70) adjacente pour réception d'une surface réfléchissante (62, 72) pouvant y être fixée.

7. Appareil formant pointe de cathéter selon la revendication 6, dans lequel ladite surface réfléchissante comprend un miroir collé dans ladite poche.

8. Appareil formant pointe de cathéter selon la revendication 6, dans lequel chacun desdits blocs verticaux (56, 66) a un alésage (58, 68) à travers pour recevoir l'une desdites fibres porteuses d'énergie.

9. Appareil formant pointe de cathéter selon la revendication 1, dans lequel ledit boîtier comprend un élément formant cadre de forme généralement cylindrique allongé (80) avec une extrémité proximale et une extrémité distale, ledit élément formant cadre ayant au moins deux paliers (84, 86) dessus d'épaisseur régressive dans la direction distale, chacun desdits paliers ayant une surface réfléchissante (88, 90) montée dessus, ladite extrémité proximale ayant une partie de tige (82) de diamètre réduit, pour permettre la réception rotative dans une gaine de cathéter.

10. Appareil formant pointe de cathéter selon la revendication 9, dans lequel ledit élément formant cadre présente un élément formant couvercle (104) agencé pour s'accoupler sur lesdits paliers (84, 86) et lesdites surfaces réfléchissantes.

11. Appareil formant pointe de cathéter selon la revendication 10, dans lequel ledit élément formant couvercle a une fente agencée de manière axiale sur celui-ci à travers une partie de sa longueur longitudinale, ladite fente étant disposée radialement adjacente à chacune desdites surfaces réfléchissantes pour permettre l'application et la collecte réfléchie d'un faisceau d'énergie à travers.

12. Appareil formant pointe de cathéter selon la revendication 9, dans lequel ladite partie de tige est fixée à un agencement de ressort hélicoïdal allongé à plusieurs couches (108) pour permettre le contrôle de torsion de ladite pointe de cathéter à l'intérieur d'un composant de corps de mammifère.

13. Appareil formant pointe de cathéter selon la revendication 1, dans lequel lesdites surfaces réfléchissantes sont des parties unitaires dudit boîtier.

14. Appareil formant pointe de cathéter selon la revendication 13, dans lequel ledit boîtier (116) a une extrémité proximale (118) et une extrémité distale (120), et ladite extrémité proximale (118) s'accouple avec une enceinte de boîtier, ladite enceinte (134) fournissant des moyens de fixation (140) pour ladite fibre de collecte d'énergie (142) et ledit boîtier (134) fournit des moyens de fixation (140) pour ladite fibre d'application (132).

15. Appareil formant pointe de cathéter selon la revendication 14, dans lequel ladite enceinte de boîtier (134) fixée à ladite extrémité proximale (118) dudit boîtier (116) a une fente allongée dirigée de manière longitudinale (144) à l'intérieur, ladite fente (144) étant en alignement radial avec lesdites surfaces réfléchissantes (122, 126) formées sur ledit boîtier (116) pour permettre la transmission et la collecte d'énergie rayonnante via lesdites surfaces réfléchissantes respectives vers un système d'analyse informatisé.

16. Appareil formant pointe de cathéter selon la revendication 1, dans lequel ledit boîtier (150) comprend un élément de forme cylindrique ayant lesdits première et seconde surfaces réfléchissantes (156, 158) usinées dessus, et dans lequel lesdites première et seconde surfaces réfléchissantes (156, 158) sont non parallèles l'une par rapport à l'autre.

17. Appareil formant pointe de cathéter selon la revendication 16, dans lequel lesdites première et seconde fibres (164, 168) sont alignées de manière diamétralement opposée l'une par rapport à l'autre autour d'un axe longitudinal de rotation dudit boîtier (150) pour minimiser l'excentricité de rotation dudit boîtier de cathéter pendant la rotation dudit boîtier (150) dans un tissu corporel.

18. Appareil formant pointe de cathéter selon la revendication 1, dans lequel ledit boîtier comprend une surface réfléchissante (198, 200) qui peut être pliée pour effectuer un changement de direction d'un faisceau d'énergie réfléchi par celle-ci.

19. Appareil formant pointe de cathéter selon la revendication 18, dans lequel ledit boîtier a des composants d'accumulation définissant une fente d'alignement de fibres (194, 196) pour miniaturiser ladite pointe.

20. Appareil formant pointe de cathéter selon la revendication 1, dans lequel ladite première fibre d'application porteuse d'énergie allongée flexible est en communication avec une source génératrice de lumière analytique contrôlée et ladite seconde fibre d'application porteuse d'énergie allongée flexible est en communication avec un dispositif d'analyse de collecte de lumière.

21. Appareil formant pointe de cathéter selon la revendication 1 ou 20,
dans lequel ladite première fibre d'application porteuse d'énergie allongée flexible (44, 98, 132, 168, 182) est une fibre conductrice de lumière en communication couplée par la lumière avec ladite première surface réfléchissante (42, 74, 90) ; et
dans lequel ladite seconde fibre de collecte porteuse d'énergie allongée flexible (30, 96, 142, 164, 184) est une fibre conductrice de lumière en communication couplée par la lumière avec ladite seconde surface réfléchissante (36, 64, 88, 122, 156, 198).

22. Appareil formant pointe de cathéter selon la revendication 1 ou 20, dans lequel ladite première surface réfléchissante (42, 74, 126, 158, 200) est dimensionnellement plus grande que ladite seconde surface réfléchissante (36, 64, 88, 122, 156, 198).

23. Appareil formant pointe de cathéter selon la revendication 1 ou 20, dans lequel ladite première surface réfléchissante (218) est curviligne.

24. Appareil formant pointe de cathéter selon la revendication 1 ou 20, dans lequel ladite première surface réfléchissante est non parallèle par rapport à ladite seconde surface réfléchissante.

25. Appareil formant pointe de cathéter selon la revendication 1 ou 20, dans lequel au moins l'une desdites première et seconde surfaces réfléchissantes sont espacées desdites fibres.

26. Appareil formant pointe de cathéter selon la revendication 1 ou 20, dans lequel ladite première surface réfléchissante est disposée radialement à l'intérieur et espacée du périmètre dudit boîtier pour permettre une propagation d'un faisceau lumineux de ladite première surface réfléchissante sur ledit tissu corporel.

27. Appareil formant pointe de cathéter selon la revendication 25, ayant un fluide d'adaptation d'indice (206) agencé entre une extrémité distale de ladite fibre (182, 184) et ladite surface réfléchissante (198, 200).

28. Appareil formant pointe de cathéter selon la revendication 25, dans lequel ladite surface réfléchissante est positionnée dans une poche de maintien (60) agencée dans ledit boîtier.

29. Appareil formant pointe de cathéter selon la revendication 28, dans lequel ladite surface réfléchissante comprend un élément miroir.

30. Appareil formant pointe de cathéter selon la revendication 28, dans lequel ladite poche de maintien (60) est utilisée pour aligner ladite surface réfléchissante par rapport audit boîtier (50).

31. Appareil formant pointe de cathéter selon la revendication 1 ou 20, dans lequel lesdites fibres conductrices de lumière sont chacune individuellement agencées à l'intérieur d'un alésage (58, 68, 92, 94, 130, 162, 166) disposé à l'intérieur dudit boîtier.

32. Appareil formant pointe de cathéter selon la revendication 1 ou 20, dans lequel lesdites fibres sont également diamétralement opposées autour d'un axe de rotation dudit boîtier pour fournir un équilibre à celui-ci et minimiser l'excentricité pendant la rotation de celui-ci.

33. Appareil formant pointe de cathéter selon la revendication 1 ou 20, dans lequel ladite première surface réfléchissante (236) et ladite seconde surface réfléchissante (238) sont disposées à un angle proportionnel à l'ouverture numérique desdites première et seconde fibres (232, 234), pour produire un faisceau lumineux avec des bords adjacents qui sont parallèles les uns aux autres, pour permettre une séparation réflecteur d'application/réflecteur de collecte indépendante de la distance.

34. Procédé d'application et de collecte d'un signal d'énergie lumineuse frappant le tissu d'un premier élément porteur de lumière et d'un élément de renvoi de faisceau d'application pour émettre le signal d'énergie lumineuse vers un tissu corporel cible, excepté si effectué sur un être vivant, et renvoyer ledit signal d'énergie lumineuse du tissu corporel cible à un élément de renvoi de faisceau de collecte adjacent à un second élément porteur de lumière pour analyse et traitement de tissu, dans un agencement porteur de lumière, comprenant les étapes consistant à : espacer ledit élément de renvoi de faisceau de collecte de manière distale dudit élément redirecteur de faisceau d'application aligné à un angle pour application et collecte de photons à partir du tissu corporel cible dans une pointe de boîtier de cathéter allongé inséré dans une gaine flexible pour insertion dans un tissu corporel de mammifère pour analyse de celui-ci, dans lequel ledit boîtier est soutenu de manière rotative à l'intérieur de la gaine, ledit boîtier ayant un axe longitudinal disposant lesdits éléments de renvoi de faisceau à un angle par rapport audit axe longitudinal dudit boîtier allongé proportionnel à une ouverture numérique desdites première et seconde fibres d'énergie.

35. Procédé selon la revendication 34, comprenant l'étape consistant à baigner lesdits réflecteurs dans un fluide d'adaptation d'indice pour minimiser la réflexion de retour dans ledit boîtier inséré dans une gaine.

36. Procédé selon la revendication 34, comprenant l'étape consistant à diriger ledit signal d'énergie lumineuse d'application et ledit signal d'énergie lumineuse de collecte de manière à obtenir des bords adjacents de celui-ci qui sont parallèles.

37. Procédé selon la revendication 34, comprenant les étapes consistant à appliquer et collecter la lumière provenant de fibres communes dans ledit agencement porteur de fibre lumineuse.

38. Procédé selon la revendication 34, dans lequel lesdites ouvertures numériques pour chacun desdits redirecteurs de faisceau sont différentes les unes des autres.

39. Procédé selon la revendication 34, dans lequel lesdits redirecteurs de faisceau sont des réflecteurs.

40. Procédé selon la revendication 34, dans lequel lesdits éléments porteurs de faisceau comprennent des fibres optiques.

41. Procédé selon la revendication 34, dans lequel lesdits éléments porteurs de faisceau comprennent des guides d'onde.
